# EUROPEAN PATENT APPLICATION

(11) **EP 4 470 373 A1**
(43) Date of publication of application: **04.12.2024**
(21) Application number: 23382527.2
(22) Date of filing: 31.05.2023
(51) Int. Cl.: A01N 1/02, A61K 35/08, A61K 9/08, A61P 1/16, A61P 3/06

(54) **POLYELECTROLYTE ISOTONIC SALINE SOLUTION FOR THE TREATMENT OF LIVER DAMAGE AND ITS USE IN ORGAN TRANSPLANTATION**

(71) Applicant: Laboratoires Quinton International, SL, 03350 Cox (ES)
(72) Inventor: Peralta Uroz, Carmen, 03350 Cox (Alicante) (ES); Gracia-Sancho, Jordi, 03350 Cox (Alicante) (ES); Gosálbez Coll, Guillermo, 03350 Cox (Alicante) (ES); Coll Sánchez, Francisco, 03350 Cox (Alicante) (ES)
(74) Representative: Sahuquillo Huerta, Jesús

(57) **Abstract**

The present invention concerns a polyelectrolyte isotonic saline solution for the treatment of liver damage and organ transplantation. More specifically, on liver injury, dysfunction, and regeneration failure in liver transplantation with steatotic and non-steatotic grafts from brain dead donors. The aqueous saline solution is preferably cold microfiltered isotonic seawater. The present invention demonstrates the usefulness of isotonic saline aqueous solution both for maintaining the functionality of liver grafts prior to implantation in the recipient and under conditions of ischemia, i.e., during transport of the organs from removal from the donor to implantation in the recipient.

## Description

### Technical field

The present invention concerns a polyelectrolyte isotonic saline solution for the treatment of liver damage and, more specifically, liver injury, dysfunction, and regeneration failure in liver transplantation with steatotic and non-steatotic grafts from brain-dead donors. The isotonic polyelectrolyte saline solution is preferably cold microfiltered isotonic seawater. The present invention demonstrates the usefulness of polyelectrolyte isotonic saline solution both for the maintenance of organ functionality prior to implantation in the recipient and under conditions of ischemia, i.e., during transport of the organs from removal from the donor to implantation in the recipient. All in accordance with the claims accompanying the present description.

### Prior art

Currently, 80% of liver grafts come from brain-dead donors. However, liver grafts from brain-dead donors have poor tolerance to preservation-reperfusion-induced injury, which negatively affects liver graft viability. In addition, a high number of livers from cadaveric donors are discarded due to excess steatosis, further aggravating the lack of liver grafts that can be used in transplantation, negatively impacting the waiting lists for liver transplantation. [Jiménez-Castro, M.B.; Gracia-Sancho, J.; Peralta, C. Brain death and marginal grafts in liver transplantation. Cell Death Dis. 2015, 6, doi:10.1038/cddis.2015.147].

Steatosis - fatty infiltration - is present in at least 50% of cadaveric donors and the presence of steatosis is a crucial factor determining post-transplant outcomes, as steatotic liver grafts are extremely vulnerable to ischemia-reperfusion (I/R) induced injury when compared to non-steatotic liver grafts. In fact, the use of steatotic liver grafts in transplantation has been associated with a high incidence of post-transplant liver graft dysfunction or primary liver graft failure [Jiménez-Castro, M.B.; Meroño, N.; Mendes-Braz, M. Gracia-Sancho, J.; Martinez-Carreres, L.; Cornide-Petronio, M.E.; Casillas-Ramirez, A.; Rodés, J.; Peralta, C. The effect of brain death in rat steatotic and non-steatotic liver transplantation with prior ischemic preconditioning. J. Hepatol. 2015, 62, 83-91, doi: 10.1016/j.jhep.2014.07.031].

In view of the above, there is a need to establish protective strategies to minimise the adverse effects produced by I/R, which is an unsolved problem in current clinical practice, with the aim of improving the post-transplant outcomes of available liver grafts, resulting in a reduction of surgical waiting lists, consequently improving the quality of life of patients once they have received the transplant. This problem is solved with the polyelectrolyte isotonic saline solution of the claims accompanying this description.

### Explanation of the invention

The present invention relates to a saline, isotonic and polyelectrolyte solution for various uses, particularly liver damage, and organ transplantation, according to the appended claims.

In a first aspect of the invention, a polyelectrolyte isotonic saline solution made from cold microfiltered seawater is claimed. In a practical example of the invention, seawater is extracted from specific areas of the Atlantic Ocean, characterised by intense biological activity, the absence of pollutants and a high concentration of nutrients. The raw material is then diluted with spring water until isotonic (salinity of 0.9%) is reached and the isotonic mixture is double microfiltered by 0.22µ m. Finally, more than 100 quality parameters are analysed, including microbiological, heavy metal, radioactivity, endotoxin, microplastic, agricultural fertiliser and other analyses.

This solution contains sodium, chloride, magnesium, calcium, potassium and bicarbonate as major elements. It also includes other chemical elements such as zinc, manganese and copper, as well as proteins, lipids, water-soluble vitamins such as D-biotin, thiamine, riboflavin, nicotinamide, cyanocobalamin and pyridoxine, and fat-soluble vitamins such as retinal, vitamin D₃, alpha-tocopherol and vitamin K₁, naturally present in the raw material in trace amounts.

In a particular embodiment of this first aspect of the invention, the polyelectrolyte isotonic saline solution for use in the treatment of liver damage can optionally be supplemented with at least one compound selected from: mannitol, lactobionate, glutathione, glutamic acid, histidine, adenosine, raffinose, tryptophan, hydroxyethyl starch (HEA), polyethylene glycol (PEG), and/or ketoglutarate.

In another embodiment, the treatment of liver damage can be in both steatotic and non-steatotic livers.

In a second aspect of the invention, a polyelectrolyte isotonic saline solution is claimed for use in the transplantation of steatotic and non-steatotic livers from cadaveric donors when the solution is applied to the donors, wherein the solution is obtained from cold microfiltered seawater and contains sodium, chloride, magnesium, calcium, potassium and bicarbonate; and further comprising at least zinc, manganese, or copper, in addition to proteins, lipids, water-soluble vitamins, and fat-soluble vitamins.

In a particular embodiment of this second aspect of the invention, the polyelectrolyte isotonic saline solution for use in the transplantation of steatotic and non-steatotic livers from cadaveric donors when the solution is applied to the donors and which can be optionally supplemented with at least one compound selected from: mannitol, lactobionate, glutathione, glutamic acid, histidine, adenosine, raffinose, tryptophan, hydroxyethyl starch (HEA), polyethylene glycol (PEG), and/or ketoglutarate.

In a third aspect of the invention, an isotonic polyelectrolyte saline solution is claimed for use in organ transplantation as a preservation solution during cold ischemia, wherein the solution is obtained from cold microfiltered seawater and contains sodium, chloride, magnesium, calcium, potassium and bicarbonate; and wherein it further comprises at least zinc, manganese or copper, as well as proteins, lipids, water-soluble vitamins and fat-soluble vitamins.

In a particular embodiment of the polyelectrolyte isotonic saline solution for use in organ transplantation as a preservation solution during cold ischemia, the solution can optionally be supplemented with at least one compound selected from: mannitol, lactobionate, glutathione, glutamic acid, histidine, adenosine, raffinose, tryptophan, hydroxyethyl starch (HEA), polyethylene glycol (PEG) and/or ketoglutarate.

In a fourth aspect of the invention, a polyelectrolyte isotonic saline solution is claimed for use in hot ischemia settings in steatotic and non-steatotic livers undergoing partial hepatectomy under vascular occlusion, wherein the solution is obtained from cold microfiltered seawater and contains sodium, chloride, magnesium, calcium, potassium and bicarbonate; and further comprising at least zinc, manganese or copper, in addition to proteins, lipids, water-soluble vitamins and fat-soluble vitamins.

In a particular embodiment of the polyelectrolyte isotonic saline solution for use in hot ischemia settings, in steatotic and non-steatotic livers undergoing partial hepatectomy under vascular occlusion, the solution may be optionally supplemented with at least one compound selected from: mannitol, lactobionate, glutathione, glutamic acid, histidine, adenosine, raffinose, tryptophan, hydroxyethyl starch (HEA), polyethylene glycol (PEG) and/or ketoglutarate.

Finally, in a fifth aspect of the invention, a polyelectrolyte isotonic saline solution is claimed for use in organ transplantation in hot ischemic contexts, wherein the solution is obtained from cold microfiltered seawater and contains sodium, chloride, magnesium, calcium, potassium, and bicarbonate; and wherein it further comprises at least zinc, manganese or copper, as well as proteins, lipids, water-soluble vitamins and fat-soluble vitamins.

In a particular embodiment of the polyelectrolyte isotonic saline solution for use in organ transplantation in warm ischaemic contexts, the solution can optionally be supplemented with at least one compound selected from: mannitol, lactobionate, glutathione, glutamic acid, histidine, adenosine, raffinose, tryptophan, hydroxyethyl starch (HEA), polyethylene glycol (PEG) and/or ketoglutarate.

Throughout the description and the claims, the word "comprises", and variants thereof are not intended to exclude other technical features, additives, components or steps. To those skilled in the art, other objects, advantages, and features of the invention will be apparent in part from the invention and in part from the practice of the invention. The following examples and drawings are provided by way of illustration and are not intended to restrict the present invention. Furthermore, the invention covers all possible combinations of particular and preferred embodiments indicated herein.

### Brief description of the drawings

The following is a very brief description of a series of drawings which help to better understand the invention, and which relate expressly to an embodiment of the invention, which is illustrated as a non-limiting example of the invention.

Transaminase levels (AST and ALT) in the study groups. *p<0.05 versus Control; +p<0.05 versus BD.
Figure 2.- HGF and VEFA levels in the study groups. *p<0.05 versus control; +p<0.05 versus BD.
Figure 3.- MDA and MPO levels in the study groups. *p<0.05 versus control; +p<0.05 versus BD.
Figure 4.- Transaminase levels (AST and ALT) in the study groups. *p<0.05 versus Group 1; ⁺p<0.05 versus Group 2.
Figure 5.- Levels of caspase 8 and caspase 9 in the study groups. *p<0.05 versus Group 1; ⁺ p<0.05 versus Group 2.
Figure 6.- MDA levels in the study groups. *p<0.05 versus Group 1; +p<0.05 versus Group 2.
Figure 7.- ATP and lactate levels in the study groups. *p<0.05 versus Group 1; ⁺ p<0.05 versus Group 2.
Figure 8.- AST and ALT levels in the study groups. *p<0.05 versus Group 7; +p<0.05 versus Group 8.

### Explanation of a detailed embodiment of the invention and examples

To investigate the effects of the described isotonic saline aqueous solution - essentially isotonic seawater - on liver injury, dysfunction, and regeneration failure in liver transplantation with steatotic and non-steatotic grafts from brain-dead donors, several experiments have been performed, as described below.

### Experimental animals

To assess the effectiveness of the aqueous solution of the invention, male rats of the Zucker strain have been used. Zucker rats are a well-established experimental model of genetically induced nutritional obesity. Zucker rats exhibit two different phenotypes: (a) homozygous (fa/fa) Zucker rats with an obese phenotype (Ob) that lack brain leptin receptors and develop obesity from 8 weeks of age due to increased food intake and decreased energy expenditure (40% - 60% steatosis); and (b) heterozygous (fa/-) Zucker rats that have brain leptin receptors and maintain a lean phenotype (Ln) throughout life. The animals will be kept in the animal house of the Faculty of Medicine of the University of Barcelona-IDIBAPS for at least two weeks prior to surgery. Environmental conditions were kept constant: temperature 21°C - 22°C, relative humidity 70% and 12-hour light-dark cycles. Animals were fed ad libitum on a diet consisting of 12% fat, 28% protein and 60% carbohydrate (5001 *rodent diet;* PMI Inc., Brentwood, MO, USA). All studies were performed in accordance with EU regulatory standards for animal models (Directive 86/609/ECC) and after approval by the ethical committee for animal experimentation of the University of Barcelona.

### Induction of brain death for steatotic and non-steatotic liver grafts

Inhalation anaesthesia with isoflurane is used and rats are mechanically ventilated using a mixture of nitrous oxide and oxygen until 30 minutes after induction of brain death (respiratory rate of 60/min). Subsequently, the rats are ventilated with 30% oxygen until the end of the procedure. Adequate ventilation of all animals is confirmed by blood gas analysis (oxygen and carbon dioxide). A frontolateral trepanation (11 mm) is then performed and a balloon catheter (Fogarty 14G, Baxter Health Care Corporation, CA) is introduced into the extradural space.

Brain death is induced by an increase in intracranial pressure by inflating the balloon for one minute, which induces rapid brain injury leading immediately to brain death, simulating a condition comparable to isolated acute brain trauma in humans. Brain death is defined by a marked increase and subsequent drop in blood pressure and heart rate. The state of brain death is confirmed by the absence of corneal reflexes and by an apnoea test. Normotension in brain dead rats - mean arterial pressure greater than 80 mm Hg - is achieved by infusion of 0.9% saline with hydroxyethyl starch, 5% and norepinephrine (0.01 mg/ml) into the tail vein (maximum flow rate 0.5 ml/min). This infusion starts 15 minutes after induction of brain death and allows for a haemodynamic stabilisation phase after brain death. Body temperature is monitored and the animal's normotension is maintained for a period of 6 hours after induction of brain death.

### Donor surgery

Once the rat is anaesthetised, a transverse laparotomy is performed 1 cm below the xiphoid appendix. The hepatic ligaments are then sectioned, and the inferior vena cava is released. Subsequently, the right renal pedicle is dissected, and the right renal artery and vein are ligated, as well as the right adrenal and lumbar veins. At the hepatic hilum, the portal vein is separated from the hepatic artery and the common bile duct. The splenic and pyloric branches of the portal vein are ligated, and a cannula is placed in the bile duct with the polyethylene catheter and secured with a double silk ligature. The right diaphragmatic vein is then released and ligated, separating the aorta from the inferior vena cava.

Once the organ is prepared for extraction, the aorta is cannulated and perfusion of the graft begins, the thoracic aorta is occluded after opening the diaphragm, the suprahepatic inferior vena cava is sectioned above the diaphragm and perfusion of the graft with preservation solution (a UW solution or Balzer's solution) begins. Once the liver is perfused, the donor liver is removed and placed in a preservation solution bath at 4°C to begin the cold ischemia period (6 hours).

Two experiments were carried out with respect to these conditions. Experiment 1 was performed to demonstrate the suitability of the isotonic aqueous saline solution of the invention for the purpose of maintaining the livers in better condition before removal from the donor. Experiment 2 demonstrates the ability to continue to protect the liver grafts during ischemia, i.e., during transport of the organs from removal from the donor to implantation in the recipient.

### Experiment 1

Subsequently, they conducted the following experimental groups:

Brain dead donors with non-steatotic livers will be maintained in normotension for 6 hours (n=8 Ln Zucker rats).

Administration of isotonic seawater to donors with non-steatotic livers just after induction of brain death and maintained in normotension for 6 hours (n=8 Ln Zucker rats).

Brain dead donors with steatotic livers will be maintained in normotension for 6 hours (n=8 Ob Zucker rats).

Administration of isotonic seawater to donors with non-steatotic livers just after induction of brain death and maintained in normotension for 6 hours (n=8 Ob Zucker rats).

After preliminary testing, to select the appropriate amount of seawater solution to be administered and the appropriate pre-treatment times to obtain the highest protection as there was no reference in the literature on this aspect, in group 1.4 donors were administered 1 ml of isotonic solution after induction of brain death, and 0.5 ml at 2h and 4h after induction of brain death. Control experiments have been performed to see if seawater has beneficial effects on the donor. To this end, in group 1.3 animals, saline was administered to donors after induction of brain death, and 0.5 ml at 2h and 4h after induction of brain death.

### Sample collection and processing

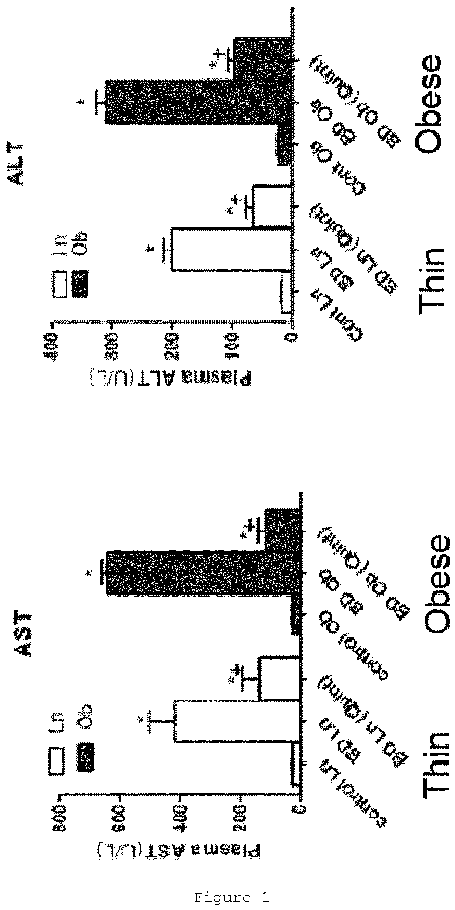

Blood and liver tissue samples were collected from brain-dead donors 6 hours after induction of brain death and prior to removal of the donor liver graft. The blood samples were centrifuged to obtain plasma and, together with the tissue samples, were frozen at -80°C until the corresponding determinations were made. Biochemical analyses of liver injury (AST, aspartate aminotransferase; ALT, alanine aminotransferase), oxidative stress and neutrophil accumulation parameters (MDA, malondialdehyde; MPO, myeloperoxidase) and cellular repair parameters (VEGFA, vascular endothelial growth factor A; HGF, hepatocyte growth factor) were performed. The results obtained are shown in the graphs in figures 1 to 3.

The administration of seawater to donors reduced liver injury, as measured by transaminase levels (ALT-AST, figure 1) in both steatotic and non-steatotic livers from cadaveric donors, indicating a protection of the marine aqueous solution of the invention already in the donor, prior to removal and implantation in the recipient.

Figure 1 shows the liver injury parameters AST on the left graph and ALT on the right graph. Ln represents mice with a lean phenotype and Ob represents obese mice with steatotic livers, i.e., with excess fat. The <Control Ln/Ob> label represents rats in normal conditions, which have been sacrificed to measure the different parameters. The <BD Ln/Ob> label is the rats after brain death, where transaminase levels are triggered and perfused with saline (current state of the art). The label <BD Ln/Ob Quinton> represents the rats after brain death, sacrificed and the different parameters measured with perfusion with the saline solution of the invention. Figure 1 shows how the results of the invention significantly improve the results of the prior art, being closer to <Control Ln/Ob> than to the results of the prior art <BD Ln/Ob>.

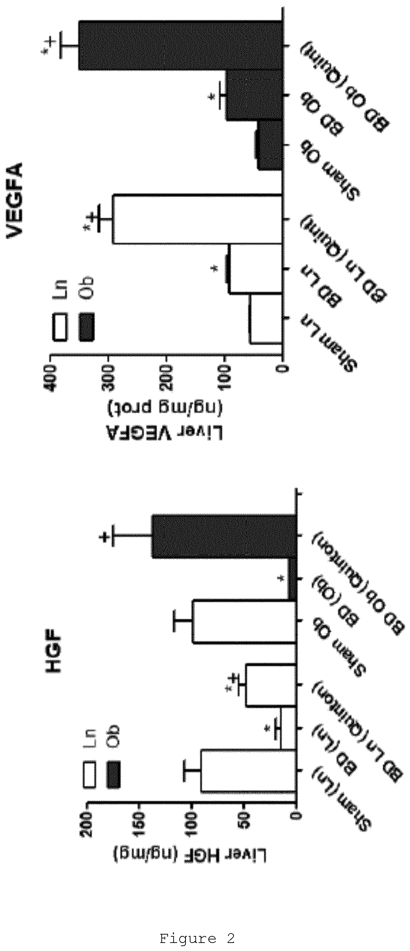

With respect to cell repair parameters, it is observed that the saline solution of the invention increased both HGF and VEGFA levels, recognised as potent mediators of cell repair in different liver disorders compared to the physiological serum currently used in the state of the art <BD Ln/Ob>, as clearly shown in figure 2.

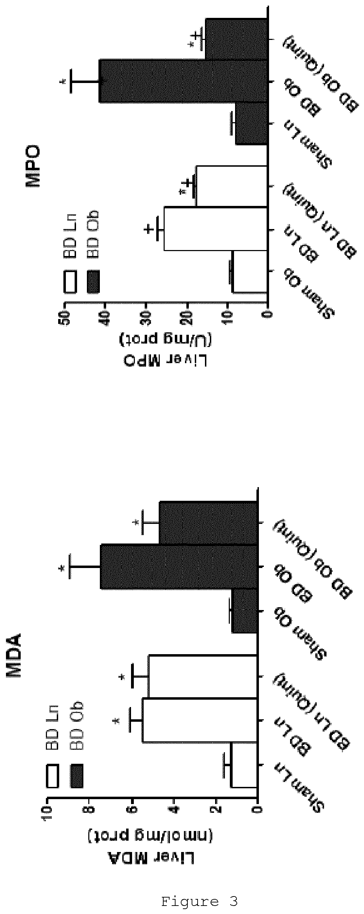

Finally, it was analysed whether the benefits of isotonic seawater could be explained by changes in oxidative stress (levels of Malondialdehyde, MDA) and neutrophil accumulation (analysed by levels of Myeloperoxidase, MPO). Figure 3 shows that the benefits of the solution of the invention (isotonic seawater) in steatotic and non-steatotic livers from cadaveric donors could not be explained by changes in oxidative stress, as no significant differences in MDA levels were observed when comparing the study groups (BD vs. BD Quinton). On the other hand, a reduction in MPO (Myeloperoxidase, which is a protein more abundant in neutrophils) levels was observed in steatotic and non-steatotic livers when treated with the isotonic saline solution of the invention, clearly indicating that this treatment reduces the accumulation of neutrophils in the liver.

The results of this first experiment are of scientific and clinical interest because, without reducing the damage to steatotic and non-steatotic donor livers before the graft is removed and placed in preservation solution for subsequent implantation in the recipient, it is difficult to obtain good post-operative outcomes after transplantation. This is not the case with many pharmacological strategies, suggesting that this treatment could be considered useful for donors to maintain the functionality of liver grafts prior to implantation in the recipient. In clinical practice, saline is known to be used before grafts are removed from the donor for implantation in the recipient. The use of the isotonic aqueous solution of the invention could replace saline to maintain the livers in better condition before they are removed from the donor. This would have a direct and favourable impact on postoperative outcomes.

### Experiment 2

This second experiment assesses whether the aqueous isotonic saline solution of the invention can continue to protect the liver grafts during ischemia, i.e., during the transport of the organs from removal from the donor to implantation in the recipient.

The following experimental groups have been carried out:

Effect of isotonic solution output on steatotic and non-steatotic livers from cadaveric donors after cold ischemia, i.e., before implantation of such grafts into the recipient.

Group 1) Brain dead donors with non-steatotic livers will be maintained in normotension for 6 hours. Donors with non-steatotic livers were given 1 ml of saline after induction of brain death, and 0.5 ml at 2h and 4h after induction of brain death. After the non-steatotic livers were maintained at normotension for 6 hours, they were perfused with Celsior solution to remove blood from within the liver and placed in a bath with Celsior preservation solution at 4°C for 6 hours (cold ischemia period) (n=6).

Group 2) Brain dead donors with steatotic livers will be maintained at normotension for 6 hours. Donors with non-steatotic livers were given 1 ml of saline after induction of brain death, and 0.5 ml at 2h and 4h after induction of brain death. After steatotic livers were maintained at normotension for 6 hours, they were perfused with Celsior solution to remove blood from within the liver and placed in a bath with Celsior preservation solution at 4°C for 6 hours (cold ischemia period) (n=6).

Group 3) Brain dead donors with non-steatotic livers will be maintained at normotension for 6 hours. Donors with non-steatotic livers were administered 1 ml of isotonic solution of the invention after induction of brain death, and 0.5 ml at 2h and 4h after induction of brain death. After the non-steatotic livers were maintained at normotension for 6 hours, they were perfused with Celsior solution to remove blood from within the liver and placed in a bath with Celsior preservation solution at 4°C for 6 hours (cold ischemia period) (n=6).

Group 4) Brain dead donors with steatotic livers will be maintained at normotension for 6 hours. Donors with steatotic livers were administered 1 ml of isotonic solution of the invention after induction of brain death, and 0.5 ml at 2h and 4h after induction of brain death. After steatotic livers were maintained at normotension for 6 hours, they were perfused with Celsior solution to remove blood from inside the liver and placed in a bath with Celsior solution at 4°C for 6 hours (cold ischemia period) (n=6).

Group 5) Brain dead donors with non-steatotic livers will be maintained at normotension for 6 hours. Donors with non-steatotic livers were administered 1 ml of isotonic solution of the invention after induction of brain death, and 0.5 ml at 2h and 4h after induction of brain death. After the non-steatotic livers were maintained at normotension for 6 hours, they were perfused with isotonic solution of the invention to remove blood from within the liver and placed in a bath with isotonic preservation solution of the invention at 4°C for 6 hours (cold ischemia period) (n=6).

Group 6) Brain dead donors with steatotic livers will be maintained at normotension for 6 hours. Donors with steatotic livers were administered 1 ml of isotonic solution of the invention after induction of brain death, and 0.5 ml at 2h and 4h after induction of brain death. After steatotic livers were maintained at normotension for 6 hours, they were perfused with isotonic solution of The Invention to remove blood from within the liver and placed in a bath with solution of The Invention at 4°C for 6 hours (cold ischemia period) (n=6).

### Sample collection and processing in Protocol 1

At the end of the protocol, samples of perfusate, preservation solution and liver tissue were collected. Samples were frozen at -80°C until the corresponding determinations were made. Biochemical analyses of necrosis-related liver injury (AST, ALT), apoptosis (caspase 8 and caspase 9), oxidative stress parameters (MDA) and energy metabolism parameters (ATP and lactate) were performed.

The results obtained are as follows:

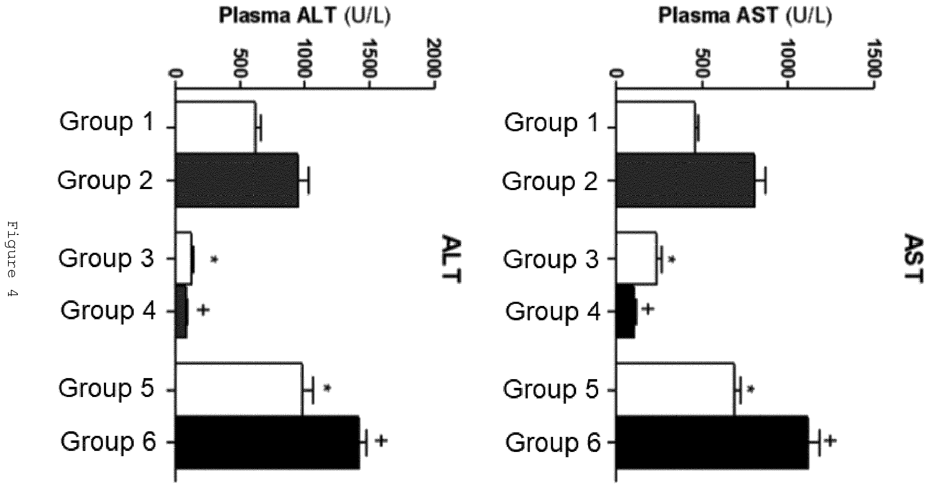

In groups 3 and 4 (steatotic and non-steatotic livers in which the solution of the invention is administered to donors and the livers are perfused with Celsior solution and preserved in Celsior solution), after 6 hours of cold ischemia, a reduction in liver injury parameters (AST, ALT) is observed compared to groups 1 and 2 (steatotic and non-steatotic livers in which saline is administered to donors and the livers are perfused with Celsior solution and preserved in Celsior solution), a reduction in liver injury parameters (AST, ALT) compared to groups 1 and 2 (steatotic and non-steatotic livers in which saline is administered to donors and livers are perfused with Celsior solution and preserved in Celsior solution). On the other hand, in groups 5 and 6 (steatotic and non-steatotic livers in which the solution of the invention is administered to donors and the livers are perfused with the solution of the invention and preserved in the solution of the invention), the levels of transaminases are higher than in the rest of the groups evaluated (groups 1-4). All this, as can be seen in figure 4.

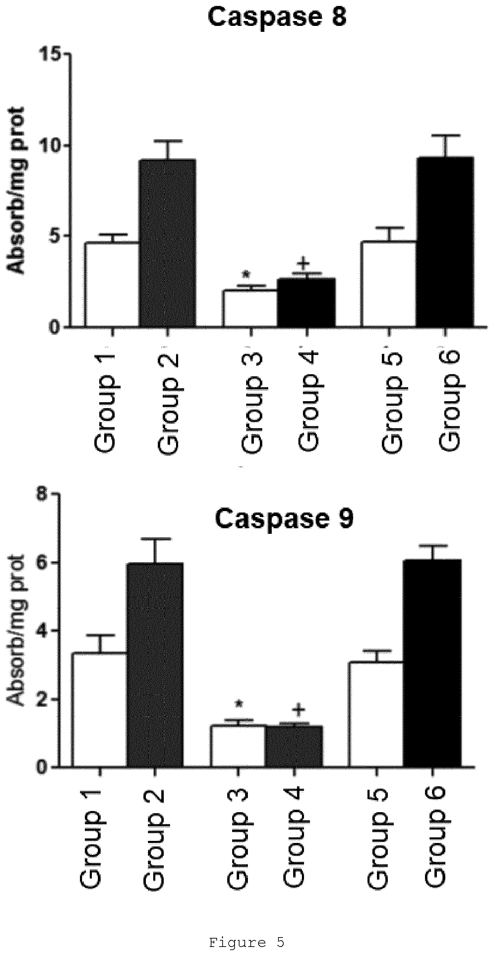

With respect to apoptosis parameters (caspase 8 and caspase 9), in groups 3 and 4 (steatotic and non-steatotic livers in which the solution of the invention is administered in donors and the livers are perfused with Celsior solution and preserved in Celsior solution), a reduction in caspase 8 and 9 levels in liver is observed after 6 hours of cold ischemia compared to groups 1 and 2 (steatotic and non-steatotic livers in which saline is administered to donors and livers are perfused with Celsior solution and preserved in Celsior solution). As with transaminases, groups 5 and 6 (steatotic and non-steatotic livers in which the solution of the invention is administered to donors and the livers are perfused with the solution of the invention and preserved in the solution of the invention), the levels of caspases are higher than in the rest of the groups evaluated (groups 1-4). This is shown in figure 5.

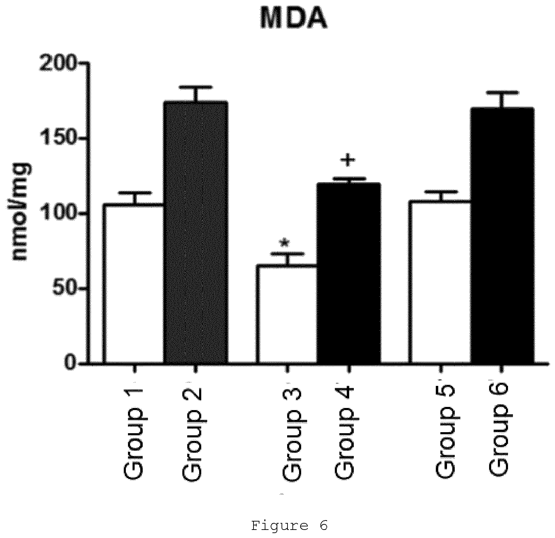

With respect to oxidative stress (MDA), in Groups 3 and 4 (steatotic and non-steatotic livers in which the solution of the invention is administered in donors and the livers are perfused with Celsior solution and preserved also in Celsior solution), a reduction in liver MDA levels is observed after 6 hours of cold ischemia with respect to Groups 1 and 2 (steatotic and non-steatotic livers in which serum is administered in Celsior solution), a reduction in liver MDA levels is observed after 6 hours of cold ischemia compared to groups 1 and 2 (steatotic and non-steatotic livers in which saline is administered to donors and livers are perfused with Celsior solution and preserved in Celsior solution). Groups 5 and 6 (steatotic and non-steatotic livers in which the solution of the invention is administered to donors and the livers are perfused with the solution of the invention and preserved in the solution of the invention) show similar MDA levels to those found in groups 1 and 2, as shown in figure 6.

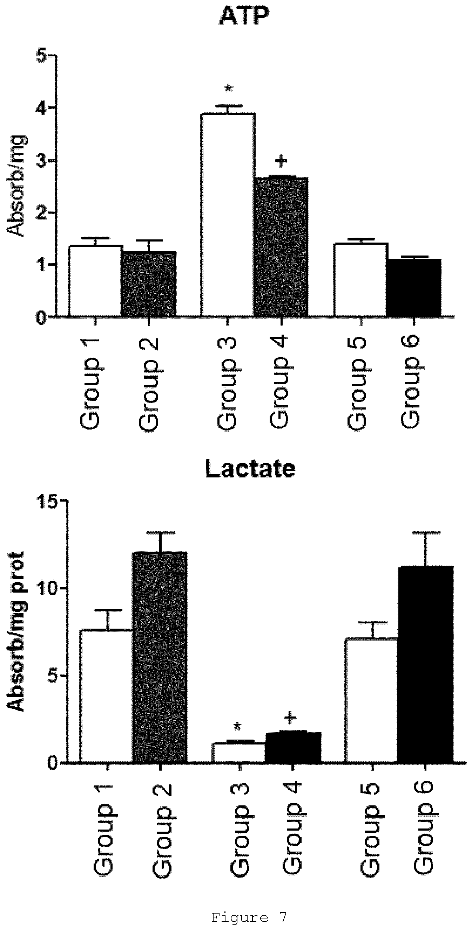

Regarding energy metabolism (ATP and lactate), ischemia degrades ATP and increases the amount of lactate (anaerobic glycolysis). In groups 3 and 4 (steatotic and non-steatotic livers in which the solution of the invention is administered in donors and the livers are perfused with Celsior solution and preserved also in Celsior solution), after 6 hours of cold ischemia, higher ATP levels and consequently a reduction in liver lactate levels are observed compared to groups 1 and 2 (steatotic and non-steatotic livers in which saline is administered to donors and livers are perfused with Celsior solution and preserved in Celsior solution). Groups 5 and 6 (steatotic and non-steatotic livers in which the solution of the invention is administered to donors and the livers are perfused with the solution of the invention and preserved in the solution of the invention) show similar ATP and lactate levels to those found in groups 1 and 2, as shown in figure 7.

Effect of the solution of the invention on steatotic and non-steatotic livers from cadaveric donors after transplantation, i.e., after the period of cold ischemia and implantation in the recipient.

Based on the results derived from protocol 1, the following experiments have been performed to assess whether the benefits observed during ischemia by the solution of the invention when administered to the donor (groups 3,4) compared to when only saline was administered (groups 1,2) were also reflected after transplantation.

The groups that took place are as follows:

Group 7) Brain dead donors with non-steatotic livers will be maintained at normotension for 6 hours. Donors with non-steatotic livers were given 1 ml of saline after induction of brain death, and 0.5 ml at 2h and 4h after induction of brain death. After the non-steatotic livers were maintained at normotension for 6 hours, they were perfused with Celsior solution to remove blood from within the liver and placed in a bath with Celsior preservation solution at 4°C for 6 hours (cold ischemia period) . After the ischemia period, the grafts will be implanted in the recipient (n=12, 6 transplants).

Group 8) Brain dead donors with steatotic livers will be maintained at normotension for 6 hours. Donors with non-steatotic livers were given 1 ml of saline after induction of brain death, and 0.5 ml at 2h and 4h after induction of brain death. After maintaining the steatotic livers in normotension for 6 hours, they were perfused with Celsior solution to remove blood from inside the liver and placed in a bath with Celsior preservation solution at 4°C for 6 hours (cold ischemia period). After the ischemia period, the grafts will be implanted into the recipient (n=12, 6 transplants).

Group 9) Brain dead donors with non-steatotic livers will be maintained at normotension for 6 hours. Donors with non-steatotic livers were administered 1 ml of isotonic solution of the invention after induction of brain death, and 0.5 ml at 2h and 4h after induction of brain death. After the non-steatotic livers were maintained at normotension for 6 hours, they were perfused with Celsior solution to remove blood from inside the liver and placed in a bath with Celsior preservation solution at 4°C for 6 hours (cold ischemia period). After the ischemia period, the grafts will be implanted in the recipient (n=12, 6 transplants).

Group 10) Brain dead donors with steatotic livers will be maintained at normotension for 6 hours. Donors with steatotic livers were administered 1 ml of isotonic solution of the invention after induction of brain death, and 0.5 ml at 2h and 4h after induction of brain death. After the steatotic livers were kept under normotension for 6 hours, they were perfused with Celsior solution to remove blood from inside the liver and placed in a bath with Celsior solution at 4°C for 6 hours (cold ischemia period). After the ischemia period, the grafts will be implanted in the recipient (n=12, 6 transplants).

### Sample collection and processing in Protocol 2

At 4 hours after liver transplantation (reperfusion time at which peaks in liver injury parameters are observed and optimal for assessing whether pharmacological strategies are protective in liver transplantation), samples were collected for biochemical analysis of liver injury (AST, ALT).

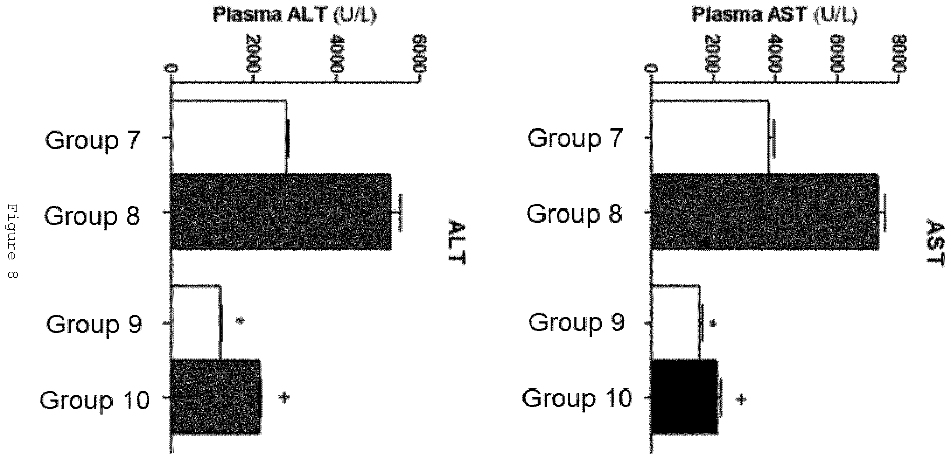

The results obtained are as follows (figure 8):

In groups 9 and 10 (steatotic and non-steatotic livers in which the solution of the invention is administered to donors and the livers are perfused in Celsior solution and preserved in Celsior solution and transplanted), a reduction in liver injury parameters (AST and ALT) is observed 4 hours after liver transplantation compared to groups 7-8 (steatotic and non-steatotic livers in which saline is administered to donors and livers are perfused with Celsior solution and preserved in Celsior solution and transplanted).

Therefore, in view of the results of experiment 2, the isotonic solution of the invention improves steatotic and non-steatotic livers against liver injury, apoptosis, oxidative stress and failure in energy metabolism when this solution is administered in the donor and another type of preservation solution (such as Celsior solution) is used during cold ischemia. Furthermore, these results are reflected post-transplantation. On the other hand, the results as preservation solution during cold ischemia are equivalent to those of preservation solutions during cold ischemia. Based on these results, the isotonic solution of the invention:
a) Improved outcomes in transplantation of steatotic and non-steatotic livers from cadaveric donors when the solution is applied to the donors; and
b) The results as an equivalent preservation solution during cold ischemia are equivalent to those of current state-of-the-art solutions (such as the Celsior solution) but it is much easier to obtain and manufacture than known solutions.

## Claims

1. An isotonic polyelectrolyte saline solution for use in the treatment of liver damage, wherein the solution is obtained from cold microfiltered seawater and contains sodium, chloride, magnesium, calcium, potassium, and bicarbonate; and further comprising at least zinc, manganese, or copper, plus proteins, lipids, water-soluble vitamins, and fat-soluble vitamins.

2. The isotonic polyelectrolyte saline solution for use in the treatment of liver damage of claim 1 which is supplemented with at least one compound selected from: mannitol, lactobionate, glutathione, glutamic acid, histidine, adenosine, raffinose, tryptophan, hydroxyethyl starch (HEA), polyethylene glycol (PEG), and/or ketoglutarate.

3. The isotonic polyelectrolyte saline solution for use in the treatment of liver damage of claim 1 or 2 for use in the treatment of liver damage in steatotic and non-steatotic livers.

4. An isotonic polyelectrolyte saline solution for use in the transplantation of steatotic and non-steatotic livers from cadaveric donors when the solution is applied to the donors, wherein the solution is obtained from cold microfiltered seawater and contains sodium, chloride, magnesium, calcium, potassium, and bicarbonate; and further comprising at least zinc, manganese, or copper, in addition to proteins, lipids, water-soluble vitamins and fat-soluble vitamins.

5. The isotonic polyelectrolyte saline solution for use in the transplantation of steatotic and non-steatotic livers from cadaveric donors when the solution is applied to the donors according to claim 4 and which is supplemented with at least one compound selected from: mannitol, lactobionate, glutathione, glutamic acid, histidine, adenosine, raffinose, tryptophan, hydroxyethyl starch (HEA), polyethylene glycol (PEG), and/or ketoglutarate.

6. An isotonic polyelectrolyte saline solution for use in organ transplantation as a preservation solution during cold ischemia, wherein the solution is obtained from cold microfiltered seawater and contains sodium, chloride, magnesium, calcium, potassium, and bicarbonate; and further comprising at least zinc, manganese, or copper, in addition to proteins, lipids, water-soluble vitamins and fat-soluble vitamins.

7. The isotonic polyelectrolyte saline solution for use in organ transplantation as a preservation solution during cold ischemia of claim 6 which is supplemented with at least one compound selected from: mannitol, lactobionate, glutathione, glutamic acid, histidine, adenosine, raffinose, tryptophan, hydroxyethyl starch (HEA), polyethylene glycol (PEG) and/or ketoglutarate.

8. An isotonic polyelectrolyte saline solution for use in hot ischaemic settings in steatotic and non-steatotic livers undergoing partial hepatectomy under vascular occlusion, wherein the solution is obtained from cold microfiltered seawater and contains sodium, chloride, magnesium, calcium, potassium, and bicarbonate; and further comprising at least zinc, manganese, or copper, plus proteins, lipids, water-soluble vitamins and fat-soluble vitamins.

9. The isotonic polyelectrolyte saline solution for use in hot ischemia settings, in steatotic and non-steatotic livers undergoing partial hepatectomy under vascular occlusion of claim 8 that is supplemented with at least one compound selected from: mannitol, lactobionate, glutathione, glutamic acid, histidine, adenosine, raffinose, tryptophan, hydroxyethyl starch (HEA), polyethylene glycol (PEG) and/or ketoglutarate.

10. An isotonic polyelectrolyte saline solution for use in organ transplantation in the context of warm ischemia, wherein the solution is obtained from cold microfiltered seawater and contains sodium, chloride, magnesium, calcium, potassium, and bicarbonate; and wherein it further comprises at least zinc, manganese, or copper, in addition to proteins, lipids, water-soluble vitamins and fat-soluble vitamins.

11. The polyelectrolyte isotonic saline solution for use in organ transplantation in the context of warm ischemia of claim 10 which is supplemented with at least one compound selected from: mannitol, lactobionate, glutathione, glutamic acid, histidine, adenosine, raffinose, tryptophan, hydroxyethyl starch (HEA), polyethylene glycol (PEG) and/or ketoglutarate.
